# EUROPÄISCHE PATENTANMELDUNG

(11) **EP 1 222 908 A2**
(43) Veröffentlichungstag der Anmeldung: **17.07.2002**
(21) Anmeldenummer: 01115454.9
(22) Anmeldetag: 27.06.2001
(51) Int. Cl.: A61F 17/00

(54) **Erste-Hilfe Kleidungsstück**

(30) Priorität: 09.01.2001 DE 10100675
(71) Anmelder: Klimmer, Uwe Rainer, 94133 Röhrnbach (DE)
(72) Erfinder: Klimmer, Uwe Rainer, 94133 Röhrnbach (DE)

(57) **Zusammenfassung**

Der Erste-Hilfe Überwurf ist kombiniert durch:
- ein reflektierendes Grundmaterial
- umlaufender und einzeln angebrachter Retroreflektions-Bänder
- eine an der Vorderseite angebrachte, transparente Fächertasche zur Unterbringung von Verbandsmaterialien.

Aufgabe der Erfindung ist, einen hohen Schutz für den Ersthelfer am Unfallort zu schaffen. Er bietet die Möglichkeit, schon beim Anlegen visuell gegenüber anderen Verkehrsteilnehmern geschützt zu sein. Der Ersthelfer hat am Unfallort beide Hände für etwaige Tätigkeiten frei. Das Verbandsmaterial liegt übersichtlich in der pro Fach einzeln verschließbaren Fächertasche.

Für alle Beteiligten ist dadurch Zeitersparnis, besseres Handling, und deutlich mehr Sicherheit gegeben.

## Beschreibung

Die Erfindung betrifft einen Erste-Hilfe Überwurf für private und gewerblich genutzte KFZ, sowie Krafträder. Der Überwurf weist mehrere Vorteile für den Ersthelfer, sowie auch für das Unfallopfer am Unfallort auf:

Ein Überwurf erlaubt dem Ersthelfer, beide Hände am Unfallort frei zu haben. Durch die Integration einer an der Vorderseite des Überwurfs angebrachten, abnehmbaren Fächertasche ( transparent ), sowie die Anbringung einer umlaufend angebrachten Retroreflektion, wird die Handlung am Unfallort wesentlich erleichtert. Zudem entfällt die Gefahr für den Ersthelfer, bei widrigen bzw. schlechten Wetterverhältnissen von anderen Verkehrsteilnehmern übersehen zu werden.

Der Ersthelfer braucht, wenn er am Ort des Unfallhergangs tätig wird, nur den Erste-Hilfe Überwurf anzulegen. Ab diesem Zeitpunkt, also noch in der sicheren Zone seines Autos, ist er gegenüber dem entgegenkommenden Verkehr schon visuell gesichert. Beim Unfallopfer hat er beide Hände frei, um evtl. Umlagerungen, etc. vorzunehmen. Dadurch, dass die Verbandsmaterialien an der Vorderseite in einer durchsichtigen Fächertasche integriert sind, entfällt das umständliche Tragen eines Verbandskastens. Zudem erlaubt die Transparenz der Fächertasche mit den verschiedenen Fächern ( einzeln zu verschließen mit Klettband ) einen raschen Überblick über die benötigten Hilfsmittel.

### Angaben zum technischen Aufbau des Erste-Hilfe Überwurfs

Der Erste-Hilfe Überwurf wird der DIN EN 471 entsprechend gefertigt. ( Zeichnung Erste-Hilfe Überwurf, Maßtabelle ) Die folgenden Herstellungs- und Qualitätsmerkmale beziehen sich auf die DIN EN 471, wobei die entsprechenden Anforderungen derselbigen im Text immer vermerkt sind. Hierbei wird vom Verfasser vermerkt, dass die Zugrundelegung der DIN-Norm lediglich der Optimierung des Produktes dient und nicht in die Patentansprüche den zu patentierbaren Anspruch einnehmen soll. Bei Erstellung des Erste-Hilfe Überwurfs diente die DIN EN 471 aufgrund der von Experten entwickelten Normen bzgl. Sicherheit im Straßenverkehr als Leitfaden.

### Definition EN 471 Warnkleidung mit hoher Sicherheit

spezifiziert die Anforderungen für *Kleidung*, *welche die Anwesenheit des Trägers visuell signalisiert mit der Absicht*, *den Träger in Gefahrensituationen unter allen Sichtbedingungen bei Tag und bei Scheinwerferlicht in der Nacht sichtbar zu machen* Technische Anforderungen werden für die folgenden Aspekte von Warnkleidung beschrieben:
**1 ) Kleidungskategorien**
**2 ) Kleidungskonzeption**
**3 ) Anforderungen an die Materialien**
**4 ) Mindestfläche**
**5 ) Physikalische Anforderungen**

### 1) Kleidungskategorien

Abhängig von der Sicherheit wird Warnkleidung in drei Kategorien unterteilt, die jeweils die Mindestflächen der zu verwendenden Untergrund- und Reflex-Materialien vorschreiben.

**Kategorie 2:** Nach dieser Kategorie richten sich die Bemessungen des Erste-Hilfe Überwurfs für DIN EN 471

Stellt den mittleren Grad dar, z.B. ärmellose Westen, Überwürfe und Latz- und Trägerhosen

### 2) Konzeption der Kleidung

### 4.2 Spezielle Anforderungen an die Konzeption

Abschnitt 4.2, in dem der Einsatzbereich für sichtbare Materialien maximiert wird, gibt vorrangig Informationen über die Plazierung und Dimensionierung reflektierender Materialien. Abgesehen von Geschirren, bei denen Bänder mit einer Mindestbreite von 30mm verwendet werden können, müssen reflektierende Bänder für Kategorie 2 eine Mindestbreite von 50mm aufweisen. ( Zeichnung Erste-Hilfe Überwurf, Punkte 2, 4, 5, 6 )

### 3 ) Anforderungen für Untergrundmaterialien

Der Absatz 3 der Norm legt folgende Definitionen für Materialien mit einzelnen Eigenschaften und kombinierten Eigenschaften fest:

### 3.1.4 Material mit einzelnen Eigenschaften

*Ein Material mit Eigenschaften des Hintergrundmaterials oder des retroreflektierenden Materials,* aber *nicht mit beiden Eigenschaften*.

Kleidungsstücke der Kategorie 2 dürfen nur aus zwei verschiedenen Materialien gefertigt werden, d.h., es muß ein Hintergrundmaterial und ein Reflektionsmaterial vorhanden sein. ( Zeichnung Erste-Hilfe Überwurf, Punkt 1, bzw. Punkte 2, 4, 5, 6 )

Je nach Gefahrengrad und entsprechender PSA stehen dem Anwender dafür die Farben gelb, orange-rot und rot zur Verfügung. ( Zeichnung erste-Hilfe Überwurf, Punkt 1 )

### 4 ) Mindestfläche an sichtbarem Material der Kategorie 2:

| | |
|---|---|
| **Untergrundmaterial** | **0,50 m**^{**2**} ( Siehe Blatt 1a, Bemaßungen ) |
| **Reflexmaterial** | **0,13 m**^{**2**} ( Siehe Blatt 1a, Bemaßungen ) |

### 5 ) Physikalische Anforderungen für Untergrund, Einzel- und Kombinationsmaterialien:

### Produktinformation Untergrundmaterial:

Artikelbeschreibung: wasserdicht, waschbar, chem. reinigungsbeständig entsprechend EN 471

### Konstruktionsmerkmale Erste-Hilfe Überwurf Untergrundmaterial:

| | | | |
|---|---|---|---|
| Materialzusammensetzung It. TKG | Textilanteil | 100% | PES |
| | Beschichtung | 100% | PUR |
| Bindung | | PES-Interlock | |
| Breite | | 150 | cm |
| Flächengewicht | | 195 | g/m |

### Technische Eigenschaften

| | | |
|---|---|---|
| Wasserdichtigkeit: | | 300mbar |
| ISO 811 | nach 5 x Wäsche | 300mbar |
| | nach 5 x chemischer Reinigung | 300mbar |
| | | |
| Höchstzugkraft: | | 650 N |
| ISO 5081 | | 250 N |
| | | |
| Maßänderungsverhalten nach 5 x Wäsche | längs | -3,0 % |
| ISO 6330 | quer | -3,0 % |
| | | |
| Maßänderungsverhalten nach 5 x chemischer Reinigung | längs | -3,0 % |
| ISO 3175 | quer | -3,0 % |

### Öko Information:

### Pestizide:

Pestizide sind auf der Ware nicht vorhanden

### Schwermetalle:

Der überwiegende Teil der eingesetzten Farbstoffe ist schwermetallfrei. Bei wenigen nicht verzichtbaren Tönen müssen jedoch Farbstoffe verwendet werden, die in gebundener Form Chrom, Kupfer, Nickel oder Kobalt in geringfügigem Umfang enthalten können. Der Schwermetallanteil liegt jedoch quantitativ unter den für Öko- Standards geforderten Höchstwerten und ist somit unbedenklich.

### Azorafarbstoffe:

Es werden grundsätzlich keine Azorafarbstoffe eingesetzt, die reduktiv Amine der MAK-Liste GR. III, A1 und A2 abspalten können.

### Färbehilfsmittel:

Carrrier auf Basis von Chloraromaten, Biphenyl- oder anderer chemischer Zusammensetzung werden grundsätzlich nicht eingesetzt.

### Echtheiten:

Die Schweiß- und Wasserechtheiten sind abhängig von Substrat, Farbstoffklasse und Farbtiefe und bewegen sich im Bereich von Note 3-5 ( nach ISO 105 E04 bzw. 105 E01 )

### Hautneutralität:

empfohlener Wert: pH 5,5 - 7,5

Durchschnittlicher Prüfwert: pH 7,0 Prüfmethode: DIN 54275 und DIN 54276

### Formaldehydgehalt:

formaldehydfrei ausgerüstet

### Reflexmaterial

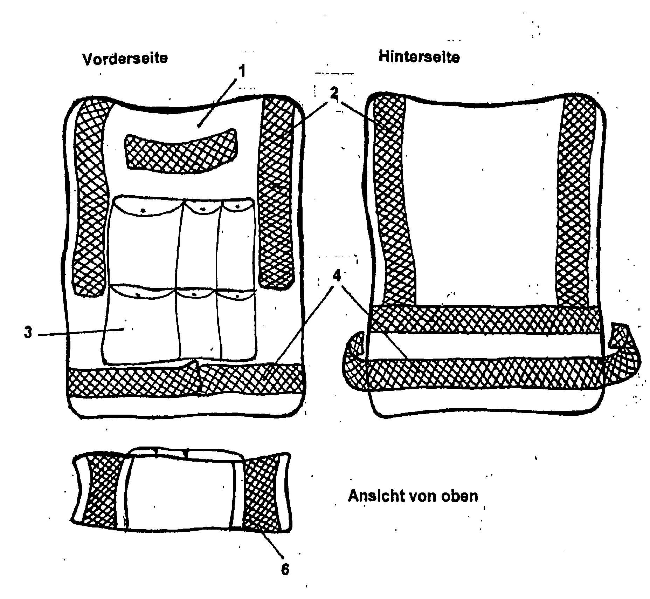

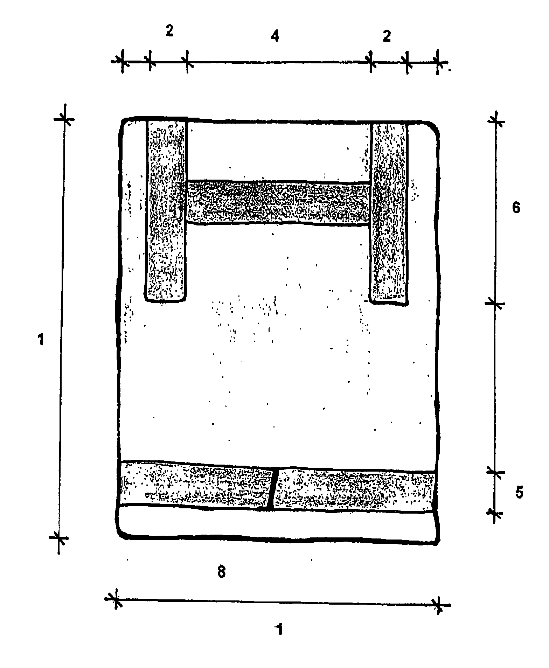

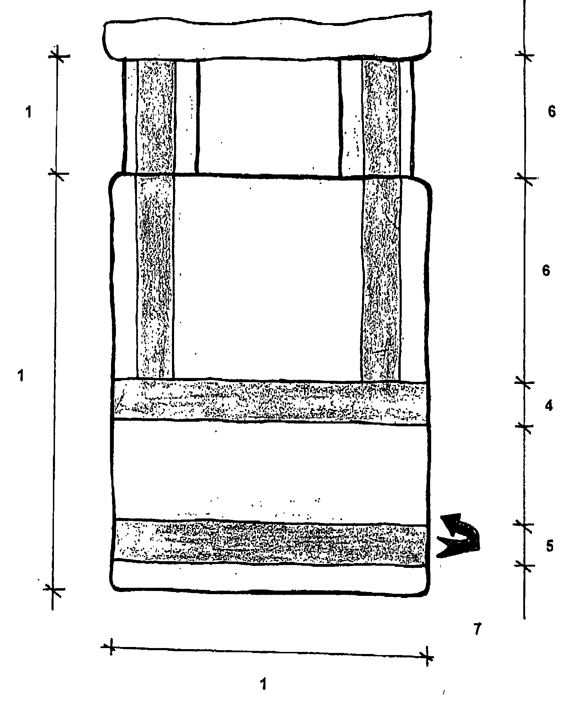

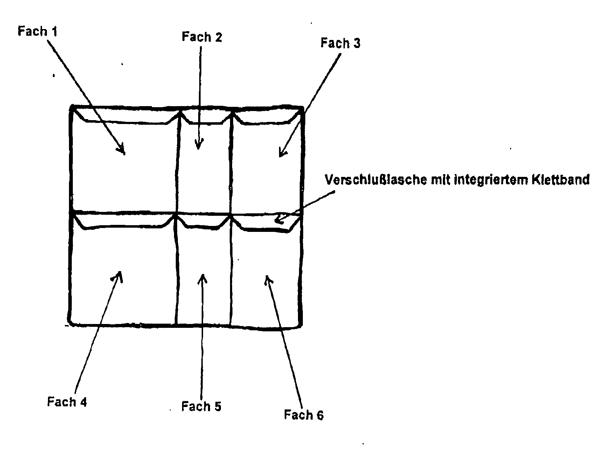

### ( Pkt. 2, 4, 5, 6 der Zeichnung Erste-Hilfe Überwurf )

Als Reflexgewebe wird **3M Scotchlite Reflexgewebe 8910** Silber verwendet. Die Rückstrahlwerte dieses Reflexgewebes liegen weit über den von der DIN EN 471, Klasse 2, geforderten. 8910 zeichnet sich durch gute Haltbarkeit ( Haushaltswäsche bis 60° C ), und Chemikalien- und Abriebbeständigkeit aus.

### 8910 Silber Bestandteile:

**Trägergewebe:**
**65% Polyester**
**35% Baumwolle**

Ich möchte Sie hier vor allem auf die Klassifizierung der DIN EN 471 in Bezug auf die Reflexmaterialien und die Hintergrundmaterialien informieren. Die optischen Anforderungen neuer Reflexmaterialien sind in Abschnitt 6 der Norm spezifiziert. Die Mindest-Rückstrahlwerte für retroreflektierendes Material der Klasse 2 finden sich in Abschn. 6, Tab.5 ( s.Abb. ) und entsprechen dem Schutzniveau der ehemaligen DIN 30711.

### Mindestkoeffizient der Reflektion in cd (lx-m² ) für Einzelmaterialien, Kategorie 2 R Kategorie 2 stellt den höchsten Rückstrahlwert dar.

| Anleuchtungswinkel | 5° | 20° | 30° | 40° |
|---|---|---|---|---|
| Beobachtungswinkel | | | | |
| 12' (0,2°) | 330 | 290 | 180 | 65 |
| 20' (0,33°) | 250 | 200 | 170 | 60 |
| 1° | 25 | 15 | 12 | 10 |
| 1° 30' (1,5°) | 10 | 7 | 5 | 4 |
| () = Werte nach DIN 30711 | | | | |

### Inhaltsliste der Fächertasche im Erste-Hilfe Überwurf

Der Inhalt ist It. 01.01.1998 durch eine neue DIN-Norm, ( DIN 13164 ), wie folgt definiert:

| **Menge** | **Erste-Hilfe Material** |
|---|---|
| 1 | Heftpflaster DIN 13019 - A 5m x 2,5cm |
| 8 | Wundschnellverbände DIN 130 - E 10 x 6 |
| 1 | Verbandpäckchen DIN 13151 - G |
| 3 | Verbandpäckchen DIN 13151 - M |
| 1 | Verbandtuch DIN 13252 - A 60cm x 80cm |
| 3 | Verbandtücher DIN 13152 - BR 40cm x 60cm |
| 6 | Kompressen 10cm x 10cm |
| 3 | Fixierbinden 8cm x 4m |
| 2 | Fixierbinden 6cm x 4m |
| 2 | Dreiecktücher DIN 13168 -D |
| 1 | 1 Erste-Hilfe Schere DIN 58279 - A 145 |
| 1 | Rettungsdecke 210 x 160cm |
| 4 | Einmalhandschuhe |
| 1 | Anleitung zur Ersten Hilfe bei Unfällen |
| 1 | Inhaltsverzeichnis |

Der Druck auf den verschiedenen Päckchen ist in deutscher Sprache. Die Beschriftung zeigt den Inhalt und alle It. Vorschrift für Verbandspäckchen notwendigen Informationen an.

### Durchsichtige Fächertasche aus Kunststoff

### ( Punkt 3, Zeichnung Erste-Hilfe Überwurf )

Der Kunststoff der Fächertasche ( PVC ) ist für den KFZ-Bedarf zugelassen. Wenn erwünscht, können die genauen Angaben hierzu nachgereicht werden.

Die Fächertasche wird an der Frontseite des Überwurfs mittels eines an der Rückseite der Fächertasche befestigten Klettverschlusses angebracht. Der Klettverschluss wird bereits beim Herstellungsprozess der Fächertasche fest mit dieser verschweisst. Durch die Befestigung mit einem Klettverschluss besteht die Möglichkeit, den Überwurf als reinen Sichtschutz zu nutzen. Dies kann zum Bsp. beim Reifenwechseln der Fall sein. ( Der Reifenwechsel, wenn sehr nah am fließenden Straßenverkehr, birgt große Gefahren, übersehen zu werden )

### Die Fächertasche ist in verschieden große Einzelfächer aufgeteilt:

### Maße entnehmen Sie bitte der Detailzeichnung Fächertasche.

Anm: Die verschiedenen Größen der Fächertasche werden in ca.-Maßen angegeben, da beim Thermo-Verschweissen geringfügig Änderungen der Größen auftreten können.

### Fach 1 : ca. 15 x 14cm, ( Punkt 3a, Zeichnung Fächertasche )

- Inhalt:: 4 Erste-Hilfe Handschuhe
1 Rettungsdecke 160cm x 210cm
1 Heftpflaster 5m x 2,5cm

### Fach 2: ca. 7,5 x 14cm , ( Punkt 3b, Zeichnung Fächertasche )

- Inhalt:: 2 Fixierbinden DIN 61634-FB 6

### Fach 3: ca. 9,5 x 14cm, ( Punkt 3c, Zeichnung Fächertasche )

- Inhalt:: 3 Fixierbinden DIN 61634-FB 8

### Fach 4: ca.15 x 15cm, ( Punkt 3d, Zeichnung Fächertasche )

- Inhalt:: 6 Päckchen Wundkompressen 10 x 10cm
2 Verbandtücher 400mm x 600mm
1 Verbandtuch 600mm x 800mm
1 Kompresse DIN 13151-G
1 Kompresse DIN 13151-M

### Fach 5: ca. 7,5 x 15cm, ( Punkt 3e, Zeichnung Fächertasche )

- Inhalt:: 2 Verbandpäckchen DIN 13151-M

### Fach 6: ca. 9,5 x 15cm, ( Punkt 3f, Zeichnung Fächertasche )

- Inhalt:: 1 Päckchen Wundschnellverband
2 Päckchen Dreieckstücher

Jedes Fach kann einzeln mittels einer die Frontseite jedes einzelnen Faches überdeckenden Lasche geschlossen sein. Die Befestigung der Lasche mit der Frontseite eines Einzelfaches geschieht mittels Klett oder Druckknopf.

Die Gesamtmaße der Fächertasche beträgt nach o. g. Angaben : Breite = 32 cm Höhe = 29cm.

### Bemaßungstabelle

### Geforderte Größe Untergrundmaterial DIN EN 471: 0,5m²

### Maße Untergrundmaterial Erste-Hilfe Überwurf:

| | |
|---|---|
| Vorderseite | h = 55cm / b = 43cm ; Gesamt = 0,2365m² |
| Hinterseite | h = 55cm / b = 43cm ; Gesamt = 0,2365m² |
| Schulterband x 2 | L = 15cm / b = 10cm ; Gesamt = 0,0300m² (2 x 0,015m²) **Gesamtfläche Untergrundmaterial: 0,503m**^{**2**} |

| **Geforderte Größe Retroreflektions-Bänder DIN EN 471: 0,13m**^{**2**} | | | |
|---|---|---|---|
| Maße Retroreflektionsbänder: | | | |
| Vorderseite: | Vorderseite: Vertikales Band links, | L = 23,5cm / b = 5cm; | Gesamt = 117,5cm² |
| | Vertikales Band rechts, | L = 23,5cm / b = 5cm; | Gesamt = 117,5cm² |
| | Horizontales Band ( Verschlussband ), | L = 36,5cm / b = 5cm; | Gesamt = 182,5cm² |
| | Horizontales Band (Verschlussband), | L = 36,5cm / b = 5cm; | Gesamt = 182,5cm² |
| | Horizontales Band ( Brusthöhe ), | L = 25,0cm/ b = 5cm; | Gesamt = 125,0cm² |
| Hinterseite: | Vertikales Band links | L = 27,0cm / b = 5cm; | Gesamt = 135,0cm² |
| | Vertikales Band rechts, | L = 27,0cm / b = 5cm; | Gesamt = 135,0cm² |
| | Horizontales Band oben, | L = 43,0cm / b = 5cm; | Gesamt = 215,0cm² |
| | Horizontales Band unten, | L = 43,0cm / b = 5cm; | Gesamt = 215,0cm² |
| Schulterband x 2: | | L = 18cm x 5cm; | Gesamt = 180cm² |
| **Gesamtfläche Retroreflektion:** | | | **0,1605m**^{**2**} |

### Legende zur Zeichnung Vorderseite-Ansicht bzw. Rückenteil-Ansicht

**1)** Untergrundmaterial Vorderteil/Rückenteil, Schulterband ( Gesamtfläche = 0,503m²)
**2)** Alle Reflektoren haben eine einheitliche Breite von 50mm
**3)** Siehe Zeichnung Fächertasche
**4)** Horizontaler Retroreflektor vorne, (125,0cm²)
**5)** Horitontales Verschlußband Vorderteil/Rückenteil, (365,0cm²)
**6)** Vertikale Retroreflektoren Vorderteil, Rückenteil, Schulterband (685,0cm²)
**7)** Pfeil deutet an, dass der untere Retroreflektor zum Verschluß beidseits nach vorne läuft
**8)** Das umlaufende Verschlußband wird an der Vordeseite mit Klettverschluß fixiert

## Patentansprüche

1. Erste-Hilfe Überwurf, welcher zwei Eigenschaften kombiniert:
1) Den Sichtschutz, gegeben durch ein reflektierendes Grundmaterial und ein retroreflektierendes Reflexmaterial.
2) Die integrierte, an der Vorderseite des Überwurfs durchsichtige Fächertasche mit dem nach DIN geforderten Inhalt eines KFZ-Verbandskastens.
